# EUROPEAN PATENT APPLICATION

(11) **EP 4 311 552 A1**
(43) Date of publication of application: **31.01.2024**
(21) Application number: 22382722.1
(22) Date of filing: 28.07.2022
(51) Int. Cl.: A61K 31/7008, A61K 31/728, A61K 31/734, A61K 31/737, A61K 45/06, A61K 9/00, A61P 1/00, A61P 1/04

(54) **GASTROINTESTINAL PROTECTANT COMPOSITION**

(71) Applicant: Bioiberica, S.A.U., 08389 Palafolls Barcelona (ES)
(72) Inventor: Sabata Ramisa, Roger, Palafolls (ES); Martínez Puig, Daniel, Palafolls (ES); Segarra López, Sergi, Palafolls (ES); Ramos Motilva, Elena, Palafolls (ES)
(74) Representative: Pons

(57) **Abstract**

The invention relates to a composition which comprises glycosaminoglycans, a polysaccharide and a monosaccharide derivative of glucose. The invention also relates to its use as a medicament and, particularly, to its use as a gastroprotector for the treatment of gastric irritation, stomach ulcers, gastroesophageal reflux disease (GERD), radiation proctitis and stomach inflammation and for the prevention of stress ulcers and to the use of composition as food supplement of complementary feed.

## Description

The invention relates to a composition which comprises glycosaminoglycans, a polysaccharide and a monosaccharide derivative of glucose. The invention also relates to its use as a medicament and, particularly, to its use as a gastroprotector for the treatment of gastric irritation, stomach ulcers, gastroesophageal reflux disease (GERD), radiation proctitis and stomach inflammation and for the prevention of stress ulcers and to the use of composition as food supplement of complementary feed.

### BACKGROUND ART

The gastrointestinal mucosal barrier is continuously exposed to noxious factors leading to the development of inflammatory, erosive, and ultimately ulcerative lesions. Non-steroidal anti-inflammatory drugs (NSAIDs) increase the incidence of gastric irritation, and the long-term use can erode or even ulcer the gastric mucosa.

Sucrose octasulfate-aluminum complex (sucralfate) has been disclosed as gastrointestinal protectant (see Marks SL, Kook PH, Papich MG, Tolbert MK, Willard MD. ACVIM consensus statement: Support for rational administration of gastrointestinal protectants to dogs and cats. J Vet Intern Med. 2018 Nov;32(6):1823-1840). Regarding its use it should be considered that aluminum absorption during sucralfate treatment is comparable to that during treatment with aluminum hydroxide, and caution should be exercised with long-term treatment in patients with renal insufficiency to avoid aluminum intoxication. Constipation, caused by aluminum hydroxide, is one of the most common adverse effects, and typically occurs in 1%-3% of human patients taking the drug. Other adverse effects in humans include xerostomia, nausea, vomiting, headache, urticaria, and rashes in 0-5% of patients (see Marks SL, Kook PH, Papich MG, Tolbert MK, Willard MD. "ACVIM consensus statement: Support for rational administration of gastrointestinal protectants to dogs and cats." J Vet Intern Med. 2018;1-18). Adverse effects in cats with chronic kidney disease has been described also (see Quimby et al. J Am Anim Hosp Assoc 2016; 52:8-12).

Accordingly, it would be desirable to have a pharmaceutical composition as gastrointestinal protectant with an improved biological effect and a better safety profile than the known gastrointestinal protector sucralfate.

### SUMMARY OF THE INVENTION

A first aspect of the present invention relates to a composition which comprises:
one or more glycosaminoglycans, preferably the glycosaminoglycans selected from chondroitin sulfate, hyaluronic acid, keratan sulfate, dermatan sulfate, heparin and heparan sulfate, more preferably the glycosaminoglycans are selected from chondroitin sulfate and hyaluronic acid;
a polysaccharide, preferably the polysaccharide is selected from sodium alginate, microcrystalline cellulose, carboxymethylcellulose sodium, calcium alginate, alginic acid, carrageenan, cassia, guar, locust bean gum and xanthan, more preferably the polysaccharide is sodium alginate; and
a monosaccharide derivative of glucose, preferably wherein the monosaccharide derivative of glucose is selected from N-acetylglucosamine (NAG), glucosamine hydrochloride and glucosamine sulfate, more preferably wherein the monosaccharide derivative of glucose is N-acetylglucosamine (NAG).

The glycosaminoglycans (GAGs) of the above defined composition are long linear polysaccharides consisting of repeating disaccharide units. The repeating two-sugar unit consists of a uronic sugar and an amino sugar, except for keratan, where in the place of the uronic sugar it has galactose, which are found in vertebrate and invertebrate animals. Several GAGs have been found in tissues and fluids of vertebrate animals. Examples of GAGs include chondroitin sulfate (which is formed by the reaction between D-glucuronic acid and N-acetylgalactosamine), keratan sulfate, dermatan sulfate, hyaluronic acid (which is naturally occurring non-sulphated glycosaminoglycan with a polymeric structure of disaccharides of N-acetyl-D-glucosamine and D-glucuronic acid), heparin and heparan sulfate.

The polysaccharides of the above defined composition act as thickeners and gelling agents. Examples include, among others, microcrystalline cellulose, carboxymethylcellulose sodium, sodium and calcium alginate (which are the sodium and calcium salts, respectively, of alginic acid, which are obtained by linear polymerization of two types of uronic acid, d-mannuronic acid and I-guluronic acid), carrageenan (which are a family of natural linear sulfated polysaccharides that are extracted from red edible seaweeds), cassia (which is a flour made from the endosperms of the seeds of *Senna obtusifolia* and *Senna tora* also called *Cassia obtusifolia* or *Cassia tora,* and which is composed of at least 75% polysaccharide, primarily galactomannan with a mannose:galactose ratio of 5:1, resulting in a high molecular mass of 200,000-300,000 Da), guar (which is a galactomannan polysaccharide extracted from guar beans), locust bean gum (which is a galactomannan vegetable gum extracted from the seeds of the carob tree) and xanthan.

The monosaccharide derivative of glucose of the above defined composition includes, among others, the naturally occurring N-acetylglucosamine, glucosamine hydrochloride and glucosamine sulfate.

In another embodiment the invention relates to the composition as defined above, which also comprises a flavor, preferably an animal origin flavor or an artificial origin flavor, more preferably an animal origin flavor or an artificial origin flavor selected from pork, beef, rabbit, horse, turkey, kidney, raw food or blood; and still more preferably artificial chicken flavor.

In another embodiment the invention relates to the composition as defined above, wherein the flavor is in a quantity from 0.1mg to 200mg/ml of total composition.

In another embodiment the invention relates to the composition as defined above, which also comprises an acidulant, preferably wherein the acidulant is selected from citric acid, sorbic acid, acetic acid, lactic acid, malic acid, tartaric acid, phosphoric acid, sodium sulfate, potassium sulfate, potassium hydrogen sulfate and calcium sulfate, and more preferably wherein the acidulant is citric acid.

In another embodiment the invention relates to the composition as defined above, which also comprises a preservative, preferably wherein the preservative is selected from sodium sorbate, calcium propionate, citric acid, propionic acid, and potassium sorbate, and more preferably wherein the preservative is potassium sorbate.

In another embodiment the invention relates to the composition as defined above, wherein:
the glycosaminoglycans are selected from chondroitin sulfate and hyaluronic acid;
the polysaccharide is sodium alginate;
the monosaccharide derivative of glucose is N-acetylglucosamine (NAG);
the flavor is artificial chicken flavor;
the acidulant is citric acid; and
the preservative is potassium sorbate.

Another aspect of the invention relates to a pharmaceutical composition which comprises the composition as defined above and one or more pharmaceutically acceptable excipients.

As indicated above, the pharmaceutical composition comprises the composition of the present invention and one or more pharmaceutically acceptable excipients. The excipients must be "acceptable" in the sense of being compatible with the other ingredients of the composition and not deleterious to the recipients thereof.

Another aspect of the invention relates to the composition as defined above or to a pharmaceutical composition as defined above, for use as a medicament, and preferably for use as a gastrointestinal protectant.

Another aspect of the invention relates to the use of the composition as defined above or to the use of the pharmaceutical composition as defined above, for the manufacture of a medicament, and preferably for the manufacture of a gastrointestinal protectant.

Another aspect of the invention relates to a method of treating and/or preventing a disease in a subject in need thereof, especially an animal or a human being, which comprises administering to said subject the composition as defined above, or the pharmaceutical composition as defined above.

Another aspect of the invention relates to the composition as defined above or to a pharmaceutical composition as defined above, for use in the treatment of a disease selected from gastric irritation, stomach ulcers, gastroesophageal reflux disease (GERD), radiation proctitis and stomach inflammation and/or in the prevention of stress ulcers.

Another aspect of the invention relates to the use of the composition as defined above or to the use of the pharmaceutical composition as defined above, for the manufacture of a medicament for the treatment of a disease selected from gastric irritation, stomach ulcers, gastroesophageal reflux disease (GERD), radiation proctitis and stomach inflammation and/or in the prevention of stress ulcers.

Another aspect of the invention relates to a method of treating a disease selected from gastric irritation, stomach ulcers, gastroesophageal reflux disease (GERD), radiation proctitis and stomach inflammation and/or of preventing stress ulcers in a subject in need thereof, especially a human being, which comprises administering to said subject the composition as defined above, or the pharmaceutical composition as defined above.

The composition of the present invention can be administered in the form of any pharmaceutical formulation, the nature of which, as it is well known, will depend upon the nature of the active compound and its route of administration. Any route of administration may be used, for example oral, parenteral, nasal, ocular, rectal and topical administration.

Particularly, the composition of present invention comprising hyaluronic acid, chondroitin sulfate, sodium alginate, N-acetylglucosamine can be administered in the form of liquid or/and powder or/and granules or/and coated granules, comprising a ratio of 1: 5.33: 20.55: 26.

Thus, in another embodiment the present invention relates to the composition as defined above, which is administered in the form of liquid or/and powder or/and granules or/and coated granules.

In another embodiment the invention relates to the composition as defined above which comprises hyaluronic acid, chondroitin sulfate, sodium alginate, N-acetylglucosamine in a ratio of 1: 5.33: 20.55: 26, respectively.

The dosage and frequency of doses will depend upon the nature and severity of the disease to be treated, the age, the general condition and body weight of the patient, as well as the particular composition administered and the route of administration, among other factors.

Accordingly, another embodiment of present invention relates to the composition as defined above which comprises the following ingredients in the following doses:
sodium alginate (15.31 mg/kg/day)
hyaluronic acid (0.75 mg/kg/day)
chondroitin sulfate (4 mg/kg/day)
N-acetylglucosamine (19.50 mg/kg/day)

The average molecular weights for hyaluronic acid and chondroitin sulfate used in the present invention may vary depending on the method of obtention. The average molecular weight for hyaluronic acid preferably is between 300,000 and 2,000,000 Dalton, more preferably between 500,000 and 1,000,000 Dalton; for chondroitin sulfate, the average molecular weight is between 5,000 and 75,000 Dalton, more preferably between 10,000 and 40,000 Dalton, and more preferably between 20,000 and 30,000 Dalton.

Thus, in another embodiment the invention relates to the composition as defined above, wherein the average molecular weight for hyaluronic acid is of between 300,000 and 2,000,000 Dalton, and preferably of between 500,000 and 1,000,000 Dalton.

In another embodiment the invention relates to the composition as defined above, wherein the average weight for chondroitin sulfate is of between 5,000 and 75,000 Dalton, preferably of between 10,000 and 40,000 Dalton, and more preferably of between 20,000 and 30,000 Dalton.

Another aspect of the invention relates to the use of the composition as defined above as food, feed, supplementary food or complementary feed.

Throughout the present specification, by the term "treatment" is meant eliminating, reducing, or ameliorating the cause or the effects of a disease. For purposes of this invention treatment includes, but is not limited to, alleviation, amelioration, or elimination of one or more symptoms of the disease; diminishment of the extent of the disease; stabilized (i.e., not worsening) state of disease; delay or slowing of disease progression; amelioration or palliation of the disease state; and remission of the disease (whether partial or total).

As used herein, "prevention" refers to preventing the occurrence of a disease in a subject that is predisposed to or has risk factors but does not yet display symptoms of the disease. Prevention includes also preventing the recurrence of a disease in a subject that has previously suffered said disease.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

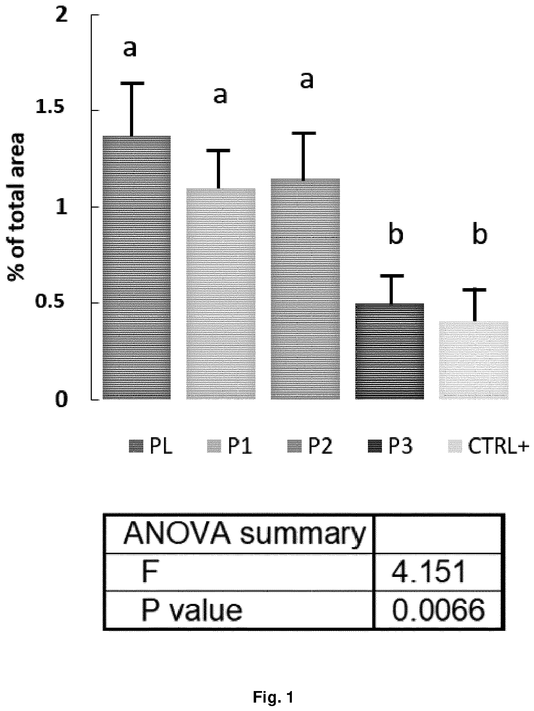

**Fig. 1****.** Histogram showing Percentage of haemorrhagic area related to the total gastric area (%). All data are presented as mean ± SEM. Post-Hoc test after ANOVA compared to PL. Different letters indicate statistically significant differences (p<0.05), wherein CTRL+ is Positive control, SEM is Standard Error Mean, F is Fisher test and P value is probability value.

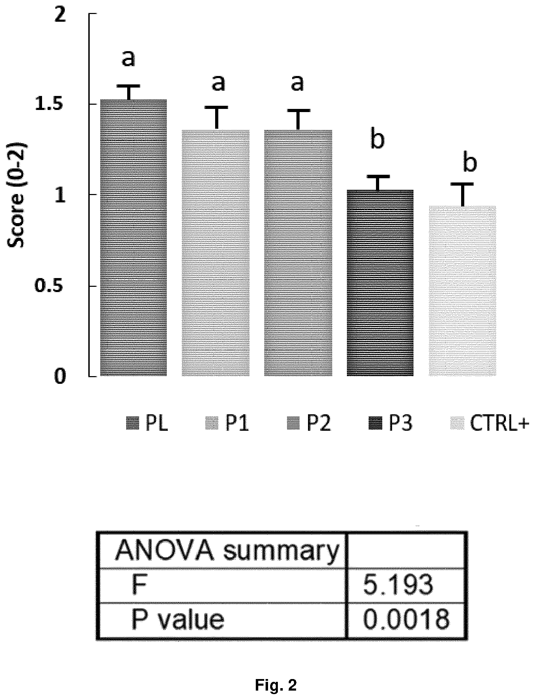

**Fig. 2****.** Histogram showing Microvascular injury. All data are presented as mean ± SEM. Post-Hoc test after ANOVA compared to PL. Different letters indicate statistically significant differences (p<0.05, wherein CTRL+ is Positive control, SEM is Standard Error Mean, F is Fisher test and P value is probability value).

### Examples

### Example 1: Obtention and Characterization of compositions prototypes P1, P2 and P3

The process to obtain the compositions of prototypes P1, P2 and P3 consist in weighting raw materials (N-acetylglucosamine, sodium alginate, artificial chicken flavor, chondroitin sulfate, potassium sorbate, hyaluronic acid, citric acid, purified water) and then adding them to the reactor, to be mixed and stirred afterwards.

| **PRODUCT** | **COMPOSITION (100mL)** | | |
|---|---|---|---|
| **PLACEBO (PL)** | 2,666.5 mg | | Xanthan gum (E-415) |
| | 200 mg | | Potassium sorbate (E-202) |
| | 20 mg | | Citric acid (E-330) |
| **Prototype 1 (P1)** | 3,852.5 mg | | Sodium alginate |
| | 1,000 mg | Chondroitin sulfate | |
| | 187.5 mg | | Sodium Hyaluronate |
| **Prototype 2 (P2)** | 4875 mg | N-acetylglucosamine | |
| | 2,666.5 mg | | Xanthan gum (E-415) |
| | 200 mg | | Potassium sorbate (E-202) |
| | 20 mg | | Citric acid (E-330) |
| **Prototype 3 (P3)** | 3,852.5 mg | | Sodium alginate |
| | 1,000 mg | Chondroitin sulfate | |
| | 187.5 mg | | Sodium Hyaluronate |
| | 4,875 mg | N-acetylglucosamine | |
| **POSITIVE CONTROL (Sucralfate) (CTRL+)** | 21,000 mg | | Sucrose octasulfate-aluminum complex (SUCRALFATE) |

### Example 2: Study of different gastroprotectors in a rat model of indomethacininduced gastric irritation.

Forty-five Sprague-Dawley rats (8-9 weeks-old, 227.6 ± 6.30g) were divided in three groups (P1, P2 and P3; n=9 each) to receive orally 0.5 mL/rat of the test treatments. Two control groups were studied as well. A negative control group that received a placebo (PL, n=9) and a positive control group (animals were administered a gastric protector used in clinical practice [sucralfate (Sucrose octasulfate-aluminum complex) (Vetgastril^{®}, Opko Health-Pharmadiet, L'Hospitalet de Llobregat, Spain)].; CTRL+, n=9). 15 minutes after the administration of the treatment, all animals were challenged with an oral administration of indomethacin (12.5 mg/kg) and sacrificed 4 hours later. Gastric mucosal injury was examined at a macroscopical and microscopical level. Macroscopic lesions were evaluated with Image J software.

Microscopically, submucosal edema and microvascular injury were scored in H&Estained preparations (Hematoxylin&Eosin-stained preparations).

The experimental protocol was approved by the Ethics Committee of the Universitat Autònoma de Barcelona (Ref: 2301-CEEA-UAB) and Generalitat de Catalunya (Ref: 11270).

Measurements were blinded and performed by two independent observers and an inter-observer mean was calculated. A significantly reduced hemorrhagic area was observed vs Placebo (p<0.05) in the prototype 3 and positive control groups (mean±SEM = 5.61±1.682 and 4.28±1.495 vs 13.05±2.700 mm2, respectively) but it was not observed in the prototype P1 and P2 groups. A significantly lower percentage of hemorrhagic area related to the total gastric area was also observed vs Placebo (p<0.05) in the prototype 3 and positive control groups (mean±SEM = 0.50±0.138% and 0.41±0.154% vs 1.37±0.260%, respectively) but it was not observed in the prototype P1 and P2 groups.

The fundic sections of the stomach were selected for microscopic evaluation since they showed evident lesions macroscopically.

Histological sections of groups Placebo, Prototype P1 and Prototype P2 showed dilated and congestive capillaries penetrating in different depths plus notable separation of the mucosa and muscular layers and significative cell loss due to marked submucosal swelling; while histological sections of groups Prototype P3 and positive control (CTRL+) showed less severe lesions, consisting of tortuous microvessels and mild to absent congestion in submucosal vessels plus moderately swelling of submucosal layer which tended to separate the mucosa and muscular layers but fairly maintained its normal aspect.

In the statistical analysis of the microscopic evaluation, significant differences were observed in Prototype 3 and positive control groups compared to the Placebo group in microvascular injury evaluation (mean±SEM = 1.03±0.077 and 0.94±0.123 vs 1.53±0.077, respectively) but it was not observed in the prototype P1 and P2 groups.

No significant statistical differences were found between prototype P3 and positive control groups. All these results showed a synergic effect between ingredients in prototype P1 and P2 that were combined in prototype P3 that was not different from the positive control (Sucralfate).

The positive control (sucralfate) is regulated as a drug, and the main advantage of the nutritional product (prototype P3) is a better safety profile.

Unexpectedly, prototype P3 has shown a superior biological effect to the other prototypes P1 and P2, and Placebo. Prototype P1 and prototype P2 were not superior to placebo group but when the active ingredients of both were combined in prototype P3 then the biological effect was superior to placebo and not different from the positive control (Sucralfate).

Moreover, the nutritional product (prototype P3) has a better safety profile compared to the drug Sucralfate.

## Claims

1. A composition which comprises:
one or more glycosaminoglycans;
a polysaccharide; and
a monosaccharide derivative of glucose.

2. The composition according to claim 1, wherein the glycosaminoglycans are selected from chondroitin sulfate, hyaluronic acid, keratan sulfate, dermatan sulfate, heparin and heparan sulfate.

3. The composition according to claim 2, wherein the glycosaminoglycans are selected from chondroitin sulfate and hyaluronic.

4. The composition according to any of claims 1 to 3, wherein the polysaccharide is selected from sodium alginate, microcrystalline cellulose, carboxymethylcellulose sodium, calcium alginate, alginic acid, carrageenan, cassia, guar, locust bean gum and xanthan.

5. The composition according to claim 4, wherein the polysaccharide is sodium alginate.

6. The composition according to any of claims 1 to 5, wherein the monosaccharide derivative of glucose is selected from N-acetylglucosamine (NAG), glucosamine hydrochloride and glucosamine sulfate.

7. The composition according to claim 6, wherein the monosaccharide derivative of glucose is N-acetylglucosamine.

8. The composition according to claim 7, which also comprises a flavor.

9. The composition according to any of claims 1 to 8, which also comprises an acidulant.

10. The composition according to any of claims 1 to 9, which also comprises a preservative.

11. The composition according to any of claims 1 to 10, wherein:
the glycosaminoglycans are selected from chondroitin sulfate and hyaluronic acid;
the polysaccharide is sodium alginate;
the monosaccharide derivative of glucose is N-acetylglucosamine (NAG);
the flavor is artificial chicken flavor;
the acidulant is citric acid; and
the preservative is potassium sorbate.

12. A pharmaceutical composition which comprises the composition of any of claims 1 to 11 and one or more pharmaceutically acceptable excipients.

13. The composition of any of claims 1 to 11 or to the pharmaceutical composition according to claim 12, for use as a medicament.

14. The composition of any of claims 1 to 11 or to the pharmaceutical composition according to claim 12, for use as gastrointestinal protectant.

15. Use of the composition according to any of claims 1 to 11 as food, food supplement, feed or complementary feed.
